# EUROPEAN PATENT APPLICATION

(11) **EP 4 830 682 A1**
(43) Date of publication of application: **28.10.2026**
(21) Application number: 24907374.3
(22) Date of filing: 16.12.2024
(51) Int. Cl.: A61G 7/018, A61B 5/11

(54) **SUPPORT SURFACE MOVEMENT CONTROL DEVICE AND SUPPORT EQUIPMENT**

(30) Priority: 18.12.2023 JP 2023212742
(71) Applicant: Minebea Mitsumi Inc., Kitasaku-gun, Nagano 3890293 (JP)
(72) Inventor: TAKAYAMA, Yoshimasa, Kitasaku-gun, Nagano 389-0293 (JP); IIDA, Norihito, Kitasaku-gun, Nagano 389-0293 (JP); ODAIRA, Masaki, Kitasaku-gun, Nagano 389-0293 (JP)
(74) Representative: Winter, Brandl - Partnerschaft mbB
(86) International application number: PCT/JP2024/044445
(87) International publication number: WO 2025/134989

(57) **Abstract**

A support surface movement control device 30 for controlling movement of a support surface 520 for supporting a body of a user U includes a body information acquisition unit 32 configured to acquire body information of the user on the support surface, and
a movement control unit 34 configured to automatically start and execute movement control to move the support surface. In the movement control, the movement control unit restricts movement of the support surface when the body information satisfies a restriction condition.

## Description

### Technical Field

The present invention relates to a support surface movement control device and support equipment.

### Background Art

As support equipment for supporting a human body, various types of equipment such as a bed, a sofa, a chair, and a wheelchair are used. In one known type of such support equipment, a support surface for supporting a human body is configured to be movable. For example, in a reclining bed, a reclining sofa, a reclining chair, or the like, a part of the support surface for supporting the upper body of a human is pivotable relative to a horizontal plane.

For the support equipment with a support surface configured to be movable, it has been proposed to control movement of the support surface by control equipment such as a computer. Patent document 1 describes a bed device including a back raising control unit for performing back raising control and a back lowering control unit for performing back lowering control.

### Citation List

### Patent Literature

Patent Document 1: JP 5665411 B

### Summary of Invention

### Technical Problem

In the equipment in the related art, it is hard to say that the safety of a user on a support surface is sufficiently secured when the support surface is moved according to control by control equipment. In addition, unnecessary movement of the support surface may be executed.

An object of the present invention is to provide a support surface movement control device and support equipment capable of moving a support surface more suitably.

### Solution to Problem

According to a first aspect of the present invention, provided is a support surface movement control device configured to control movement of a support surface for supporting a body of a user, the support surface movement control device including,
a body information acquisition unit configured to acquire body information of the user on the support surface, and
a movement control unit configured to automatically start and execute movement control to move the support surface, and
in the movement control, the movement control unit restricts movement of the support surface when the body information satisfies a restriction condition.

According to a second aspect of the present invention, provided is support equipment including,
a support surface configured to support a body of a user, and
the support surface movement control device according to the first aspect.

### Advantageous Effects of Invention

According to the support surface movement control device and the support equipment of the present invention, the support surface can be moved more suitably.

### Brief Description of Drawings

FIGS. 1(a) to 1(c) are side views of a bed, and the bed is a control target of a bed plate movement control device. In FIG. 1(a), a first bed plate, a second bed plate, a third bed plate, and a fourth bed plate are in a horizontal position. In FIG. 1(b), the first bed plate is in a back-up position, and the second bed plate, the third bed plate, and the fourth bed plate are in the horizontal position. In FIG. 1(c), the first bed plate is in the back-up position, the second bed plate is in the horizontal position, and the third bed plate, the fourth bed plate are in a knee-up position.
FIG. 2 is a plan view illustrating an arrangement of load detectors with respect to a bed.
FIG. 3 is a block diagram illustrating a configuration of a bed plate movement control system according to an embodiment of the present invention.
FIG. 4 is a flowchart of a step of acquiring body information.
FIG. 5(a) is an explanatory view illustrating vibration of the center of gravity according to respiration of a user. FIG. 5(b) is an explanatory diagram illustrating the direction of the body axis of the user in a decubitus position. FIG. 5(c) is an explanatory diagram illustrating the direction of the body axis of the user in a sitting-up posture. FIG. 5(d) is an explanatory diagram illustrating the direction of the body axis of the user in an edge sitting position.
FIGS. 6(a), 6(b), and 6(c) are graphs showing changes in a load signal depending on a lying posture of the user. FIG. 6(a) shows change in a load signal when the user is in a supine position, FIG. 6(b) shows change in the load signal when the user is in a lateral position (lateral decubitus position), and FIG. 6(c) shows change in the load signal when the user is in a prone position (abdominal position).
FIG. 7 is a flowchart of a step of acquiring biological information.
FIG. 8 is a flowchart of wake-up movement control.
FIG. 9 is a flowchart of a movement start control step.
FIG. 10 is a flowchart of an in-motion control step.
FIG. 11 is a flowchart of falling-asleep movement control.

### Description of Embodiments

### Embodiments

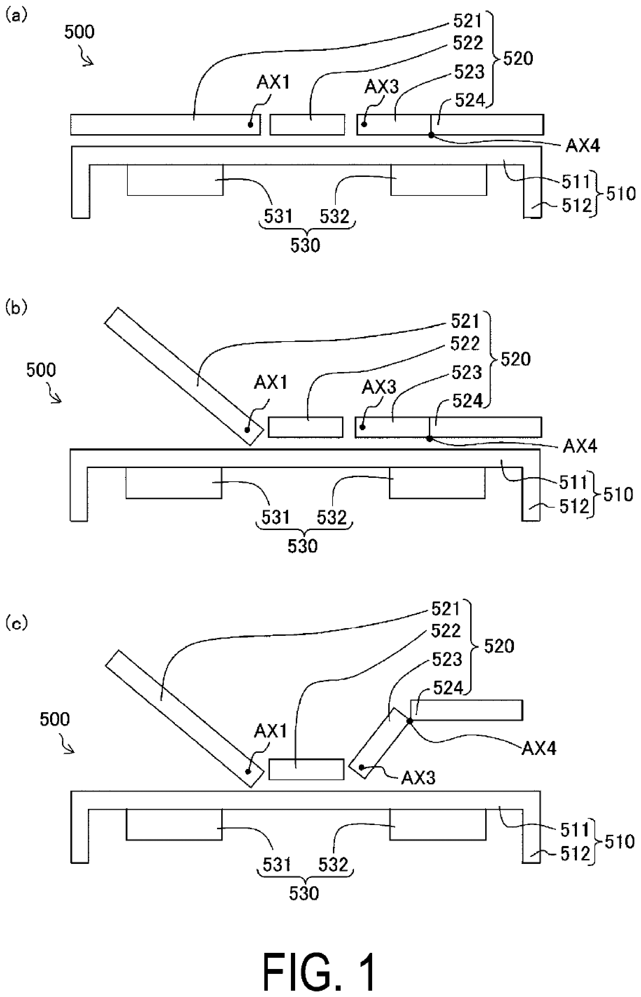

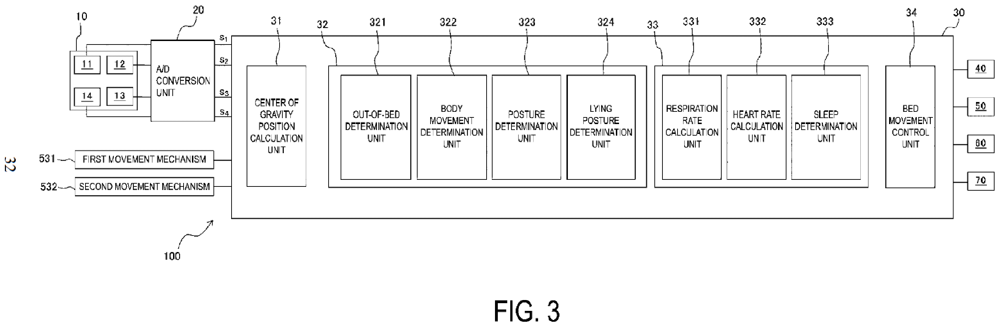

A bed plate movement control system 100 (FIG. 3) of an embodiment of the present invention will be described with reference to FIGS. 1 to 11, using, as an example, a case of the bed plate movement control system 100 used in a bed 500 (FIG. 1. An example of "support equipment").

### Bed 500

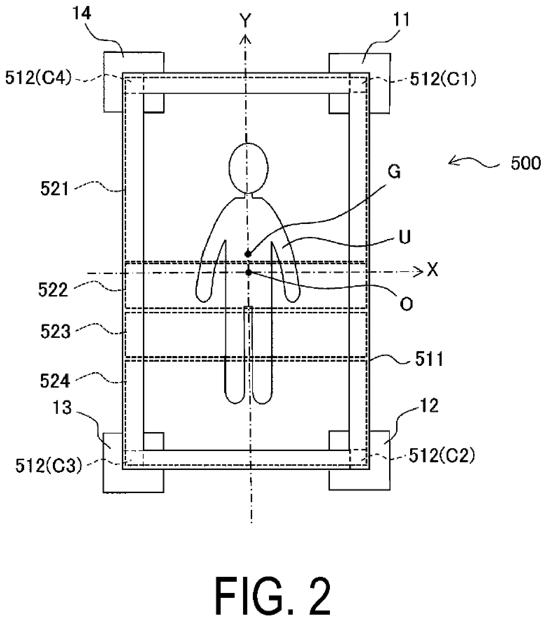

As illustrated in FIGS. 1 and 2, the bed 500 controlled by the bed plate movement control system 100 includes a base part 510, a bed plate 520 (an example of a "support surface") supported by the base part 510, and a movement mechanism 530 for moving the bed plate 520. In the following description, a long side direction (a direction of a Y axis in FIG. 2) and a short side direction (a direction of an X axis in FIG. 2) of the bed 500 and the bed plate 520 are referred to as a longitudinal direction and a width direction of the bed 500 and the bed plate 520, respectively.

The base part 510 includes a frame 511 having a rectangular shape in a plan view, and four leg parts 512 provided at four corners of the frame 511.

The bed plate 520 includes a first bed plate 521, a second bed plate 522, a third bed plate 523, and a fourth bed plate 524 (each of the first bed plate 521, the second bed plate 522, the third bed plate 523, and the fourth bed plate 524 is an example of "a part of the support surface"), in order from a head side of the bed 500 (the positive side in the Y-axis direction in FIG. 2), along the longitudinal direction of the bed 500. Generally, the first bed plate 521, the second bed plate 522, the third bed plate 523, and the fourth bed plate 524 respectively support the upper body, the buttocks, the thighs, and the lower legs of a user U (FIG. 2) on the bed plate 520.

The first bed plate 521 is pivotable about an axis AX1 located near the second bed plate 522 and extending in the width direction of the bed 500. The second bed plate 522 is fixed to the base part 510. The third bed plate 523 is pivotable about an axis AX3 located near the second bed plate 522 and extending in the width direction of the bed 500. The fourth bed plate 524 is connected to the third bed plate 523 and is pivotable about an axis AX4 located at a connection point with the third bed plate 523 and extending in the width direction of the bed 500.

The movement mechanism 530 includes a first movement mechanism 531 for moving the first bed plate 521 and a second movement mechanism 532 for moving the third bed plate 523 and the fourth bed plate 524. In the present embodiment, the first movement mechanism 531 and the second movement mechanism 532 are each an electric cylinder. However, the first movement mechanism 531 and the second movement mechanism 532 may be any actuators.

Operation of the first movement mechanism 531 causes the first bed plate 521 to pivot about the axis AX1 and displace between a horizontal position (FIG. 1(a)) with a top surface of the first bed plate 521 coinciding with the horizontal plane and a back-up position (FIGS. 1(b), 1(c)) where the top surface of the first bed plate 521 is inclined with respect to the horizontal plane. In the horizontal position, the top surface of the first bed plate 521 and a top surface of the second bed plate 522 are flush with each other.

Operation of the second movement mechanism 532 causes the third bed plate 523 to pivot about an axis AX3. At this time, the fourth bed plate 524 pivots about the axis AX4, and moves in a vertical direction while maintaining coincidence between a top surface of the fourth bed plate 524 and the horizontal plane. As a result, the third bed plate 523 and the fourth bed plate 524 are displaced between a horizontal position (FIGS. 1(a), 1(b)) with a top surface of the third bed plate 523 and top surface of the fourth bed plate 524 coinciding with the horizontal plane and a knee-up position (FIG. 1(c)) where the top surface of the third bed plate 523 is inclined with respect to the horizontal plane and the top surface of the fourth bed plate 524 coincides with the horizontal plane and is located above the top surface of the second bed plate 522. In the horizontal position, the top surface of the third bed plate 523 and the top surface of the fourth bed plate 524 are flush with the top surface of the second bed plate 522.

### Configuration of Bed Plate Movement Control System 100

As illustrated in FIG. 3, the bed plate movement control system 100 of the present embodiment mainly includes a load detection unit 10, a control unit 30 (an example of a support surface movement control device), and a storage unit 40. The load detection unit 10 and the control unit 30 are connected via an A/D conversion unit 20. A display unit 50, a notification unit 60, and an input unit 70 are further connected to the control unit 30. The first movement mechanism 531 and the second movement mechanism 532 of the bed 500 are connected to the control unit 30.

The load detection unit 10 includes four load detectors 11, 12, 13, and 14. Each of the load detectors 11, 12, 13, and 14 is a load detector for detecting a load by using, for example, a beam type load cell. The load detectors 11, 12, 13, and 14 are each connected to the A/D conversion unit 20 by wiring or wirelessly.

As illustrated in FIG. 2, the four load detectors 11 to 14 of the load detection unit 10 are respectively arranged under casters C1, C2, C3, and C4 attached to lower end parts of four legs 512 of the bed 500.

The A/D conversion unit 20 includes an A/D converter for converting an analog signal from the load detection unit 10 into a digital signal, and is connected to each of the load detection unit 10 and the control unit 30 by wires or wirelessly.

The control unit 30 is a dedicated or general-purpose computer, and a center of gravity position calculation unit 31, a body information acquisition unit 32, a biological information acquisition unit 33, and a bed movement control unit 34 (an example of a movement control unit) are built into an interior of the control unit 30. The body information acquisition unit 32 includes an out-of-bed determination unit 321, a body movement determination unit 322, a posture determination unit 323, and a lying posture determination unit 324. The biological information acquisition unit 33 includes a respiration rate calculation unit 331, a heart rate calculation unit 332, and a sleep determination unit 333.

The storage unit 40 is a storage device for storing data used in the bed plate movement control system 100, and, for example, a hard disk (magnetic disk) can be used. The display unit 50 is a monitor, such as a liquid crystal monitor, for displaying information output from the control unit 30. The notification unit 60 includes a device, such as a speaker, for audibly providing a predetermined notification based on information from the control unit 30. The input unit 70 is an interface for performing a predetermined input to the control unit 30 and can be a keyboard and a mouse.

### Operation of Bed Plate Movement Control System 100

The bed plate movement control system 100 constantly acquires body information and biological information of the user U on the bed 500, and automatically moves the bed plate 520 by control based on the acquired information. Hereinafter, acquisition of body information, acquisition of biological information, and bed plate movement control based on the body information and the biological information will be described in this order.

### Acquisition of Body Information

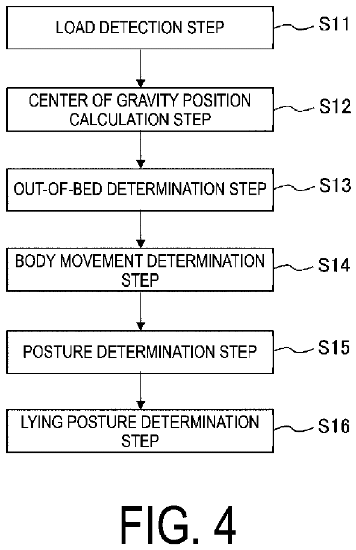

The acquisition of body information is executed by the body information acquisition unit 32 of the control unit 30. The body information acquisition unit 32 of the present embodiment continuously acquires the body information of the user U on the bed 500, at a predetermined cycle. As illustrated in a flowchart of FIG. 4, the acquisition of body information executed by the body information acquisition unit 32 mainly includes a load detection step S11, a center of gravity position calculation step S12, an out-of-bed determination step S13, a body movement determination step S14, a posture determination step S15, and a lying posture determination step S16.

In the load detection step S11, the load detectors 11, 12, 13, and 14 are used to detect the load of the user U on the bed plate 520 (the bed 500). The load of the user U on the bed plate 520 is dispersedly applied to and dispersedly detected by the load detectors 11 to 14 arranged below the four legs 512 of the bed 500.

The load detectors 11 to 14 each detect loads (load changes) and output the loads to the A/D conversion unit 20 as analog signals. The A/D conversion unit 20 converts the analog signals into digital signals with a sampling period of, for example, 5 milliseconds, and outputs the digital signal (hereinafter, referred to as a "load signal") to the control unit 30. Hereinafter, the load signals acquired by digitally converting the analog signals output from the load detectors 11, 12, 13, and 14 at the A/D conversion unit 20 are referred to as load signals s₁, s₂, s₃, and s₄, respectively.

In the center of gravity position calculation step S12, the center of gravity position calculation unit 31 calculates the position of a center of gravity G of the user U, based on the load signals s₁, s₂, s₃, and s₄.

The position of the center of gravity G is calculated by the following (Equation 1) and (Equation 2), and in (Equation 1) and (Equation 2), (x, y) is the position of the center of gravity G in the XY coordinates illustrated in FIG. 2, (X₁, Y₁), (X₂, Y₂), (X₃, Y₃), and (X₄, Y₄) are the coordinates of the load detectors 11, 12, 13, and 14, respectively, and W₁, W₂, W₃, and W₄ are partial loads of the user U indicated by the load signals si, s₂, s₃, and s₄, respectively.
[Mathematical Expression 1] $x = \frac{{X}_{1} \times {W}_{1} + {X}_{2} \times {W}_{2} + {X}_{3} \times {W}_{3} + {X}_{4} \times {W}_{4}}{{W}_{1} + {W}_{2} + {W}_{3} + {W}_{4}}$ (EQUATION 1)
[Mathematical Expression 2] $y = \frac{{Y}_{1} \times {W}_{1} + {Y}_{2} \times {W}_{2} + {Y}_{3} \times {W}_{3} + {Y}_{4} \times {W}_{4}}{{W}_{1} + {W}_{2} + {W}_{3} + {W}_{4}}$ (EQUATION 2)

In the out-of-bed determination step S13, the out-of-bed determination unit 321 determines whether the user U is out of the bed 500, based on the load applied to the bed plate 520 by the user U. Specifically, for example, the out-of-bed determination unit 321 calculates the stun of the partial loads W₁, W₂, W₃, and W₄, and determines that the user U is out of the bed 500 when the calculated value (hereinafter referred to as "user load W") is smaller than a threshold value TH_{W}. The out-of-bed determination unit 321 may perform out-of-bed determination based on the center of gravity G of the user U calculated in the center of gravity position calculation step S12, instead of or in addition to the out-of-bed determination based on the user load W. Specifically, for example, when the center of gravity G is not calculated, it may be determined that the user U is out of the bed 500.

In the body movement determination step S14, the body movement determination unit 322 determines whether body movement of the user U is occurring, based on at least one of the load signals s₁, s₂, s₃, or s₄.

This "body movement" means movement of the head, torso (trunk), and four limbs of the user. The body movement does not include the movement of organs, blood vessels, and the like associated with respiration, heartbeat, and the like. As an example, the body movement can be classified into large body movement involving movement of the torso (trunk) of the user U and small body movement involving only movement of the four limbs and the head of the user. An example of the large body movement is turning over, getting up, or the like, and an example of the small body movement is movement of the limbs, head, or the like during sleep.

Variation of the sampling value of the load signal s₁ from the load detector 11 is small in the period of no body movement of the user U, and is large in the period of movement of the user U. The same applies to the load signals s₂, s₃, and s₄ from the load detectors 12, 13, and 14.

Thus, the body movement determination unit 322 calculates standard deviation representing the magnitude of variation of the sampling value in the predetermined period (five seconds, for example) for at least one of load signals s₁ to s₄ from the load detectors 11 to 14, and determines whether body movement of the user U is occurring based on comparison between the calculated standard deviation and a predetermined threshold value TH .

Specifically, for example, when the value of the standard deviation calculated for a predetermined period is smaller than the predetermined threshold value TH , body movement of the user U is determined to be not occurring in that period. On the other hand, when the value of the standard deviation calculated for a predetermined sampling period is equal to or larger than the predetermined threshold value TH , body movement of the user U is determined to be occurring in that period. Note that, instead of the standard deviation , variance ² may be compared with a predetermined threshold value to determine whether body movement of the user U is occurring.

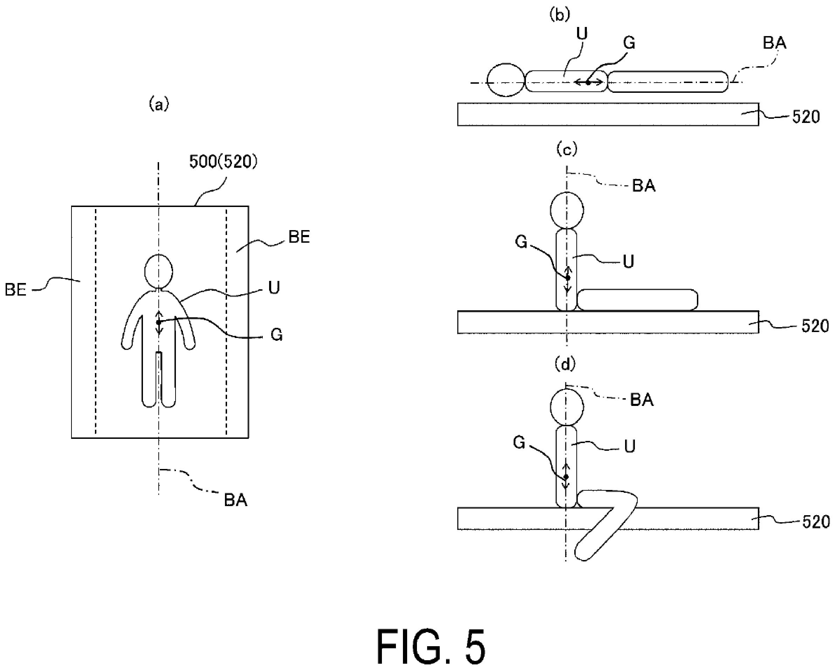

In the posture determination step S15, the posture determination unit 323 determines the posture of the user U on the bed plate 520. In the present embodiment, the posture determination unit 323 determines whether the posture of the user U is a decubitus position (a posture of lying on the bed plate 520. FIG. 5(b)), a sitting-up posture (a posture assumed when the upper body is raised on the bed plate 520. FIG. 5(c)), or an edge sitting position (a posture of sitting on an edge part of the bed plate 520. FIG. 5(d)).

In the present embodiment, the posture determination unit 323 determines the posture of the user U, based on the following principle.

When a human performs respiration, the position of the center of gravity of the human vibrates in the direction of the spine of the human, due to movement of the diaphragm and accompanying movement of the internal organs. Specifically, the center of gravity moves to the head side according to exhalation, and moves to the leg side according to inhalation. Thus, as illustrated in FIG. 5(a), the position of the center of gravity G of the user U vibrates along the spine (a body axis BA) of the user U, according to the respiration of the user U.

Here, when the posture of the user U on the bed plate 520 is the decubitus position (FIG. 5(b)), the extending direction of the body axis BA of the user U is an in-plane direction of the top surface of the bed plate 520, and thus the amplitude of the vibration of the center of gravity G calculated in the center of gravity position calculation step S12, according to the respiration of the user U, is relatively large. On the other hand, when the posture of the user U on the bed plate 520 is the sitting-up posture (FIG. 5(c)) or the edge sitting position (FIG. 5(d)), the extending direction of the body axis BA of the user U is a direction intersecting the top surface of the bed plate 520, and thus the amplitude of the vibration of the center of gravity G calculated in the center of gravity position calculation step S12, according to the respiration of the user U, is small.

Thus, the posture determination unit 323 determines that the user U is in the decubitus position, when an amplitude AM of the vibration along a predetermined direction over time, in a trajectory of the center of gravity G calculated in the center of gravity position calculation step S12, is larger than a predetermined threshold value TH_{AM}, and determines that the user U is in the sitting-up posture or the edge sitting position, when the amplitude AM is equal to or smaller than the predetermined threshold value TH_{AM}.

When determining that the user U is in the sitting-up posture or the edge sitting position, the posture determination unit 323 further determines whether the center of gravity G of the user U is located within the bed edge region BE (FIG. 5(a)). The posture determination unit 323 determines that the posture of the user U is the edge sitting position when determining that the center of gravity G of the user U is located within the bed edge region BE, and determines that the posture of the user U is the sitting-up posture when determining that the center of gravity G of the user U is not located within the bed edge region BE. The bed edge region BE may be regions on the bed plate 520 within a predetermined distance from both sides in the width direction.

Note that, when the user U changes from the decubitus position to the sitting-up posture, the upper body of the user U moves to the leg side of the bed plate 520 (the negative side in the Y direction), and thus the position of the center of gravity G moves to the leg side of the bed plate 520 (the negative side in the Y direction) along the body axis BA. Conversely, when the posture of the user U on the bed plate 520 changes from the sitting-up posture to the decubitus position, the upper body of the user U moves to the head side of the bed plate 520 (the positive side in the Y direction), and thus the position of the center of gravity G moves to the head side of the bed plate 520 (the positive side in the Y direction) along the body axis BA. The direction of the body axis AX of the user U can be estimated based on the direction of vibration over time (i.e., the direction of vibration according to the respiration) in the trajectory of the center of gravity G.

Thus, in a state of the posture of the user U being determined to be the decubitus position, when the center of gravity G of the user U moves to the leg side of the bed plate 520 along the body axis BA by more than a predetermined distance within a predetermined period, the posture determination unit 323 may determine that the posture of the user U has changed to the sitting-up posture. Further, in a state of the posture of the user U being determined to be the sitting-up posture, when the center of gravity G of the user U moves to the head side of the bed plate 520 along the body axis BA by more than a predetermined distance within a predetermined period, the posture determination unit 323 may determine that the posture of the user U has changed to the decubitus position.

In the lying posture determination step S16, the lying posture determination unit 324 determines lying posture of the user U on the bed plate 520. When the posture of the user U is determined to be the decubitus position in the posture determination step S15, the lying posture determination unit 324 determines whether the lying posture of the user U is a supine position, a lateral position (lateral decubitus position), or a prone position (abdominal position).

In the present embodiment, the lying posture determination unit 324 determines the lying posture of the user U, based on the following principle.

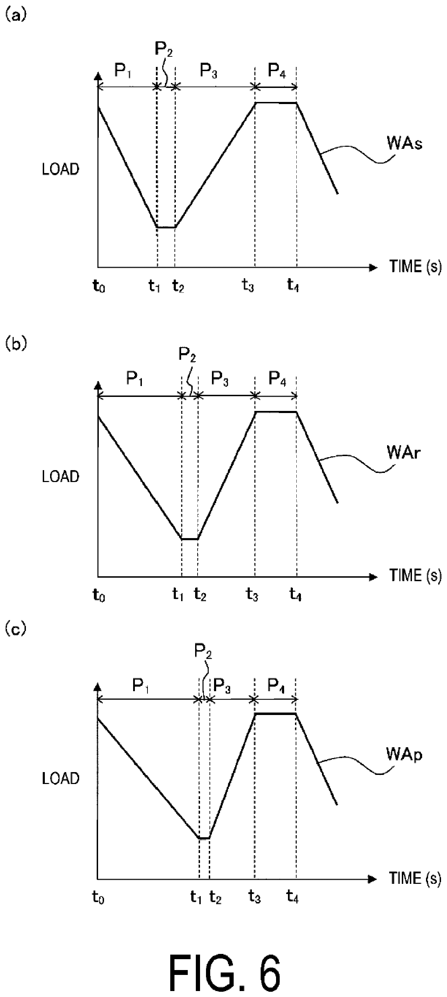

FIGS. 6(a), 6(b), and 6(c) each show waveform of a fluctuation according to the respiration of the user U, in the load signal s₁ from the load detector 11 arranged at the head side of the bed plate 520. FIG. 6(a) shows a waveform when the lying posture of the user U is the supine position ("supine position waveform WAs"), FIG. 6(b) shows a waveform when the lying posture of the user U is the lateral position ("lateral position waveform WAr"), and FIG. 6(c) shows a waveform when the lying posture of the user U is the prone position ("prone position waveform WAp").

In all of the supine position waveform WAs, the lateral position waveform WAr, and the prone position waveform WAp, the load signal s₁ gradually decreases during a period from time t₀ to time t₁ (inhalation period P₁₎ being a period of inhalation performed by the user U, and the load signal s₁ gradually increases during a period from time t₂ to time t₃ (exhalation period P₃) being a period of exhalation performed by the user U. This is because the center of gravity G moves away from the load detector 11 during inhalation and the center of gravity G moves toward the load detector 11 during exhalation. The load signal s₁ is substantially constant during a period from finish of inhalation of the user U at time t₁ to start of exhalation at time t₂ (post-inhalation hold period P₂) and during a period from finish of exhalation of the user U at time t₃ to disclosure of inhalation at time t₄ (post-exhalation hold period P₄).

Here, in the prone position waveform WAp, the inhalation period P₁ is longer than the inhalation period P₁ of the supine position waveform WAs, the post-inhalation hold period P₂ is shorter than the post-inhalation hold period P₂ of the supine position waveform WAs, and the exhalation period P₃ is shorter than the exhalation period P₃ of the supine position waveform WAs. In other words, in the shape of the prone position waveform WAp, the lowest point at the valley side is shifted in the positive direction of the time axis as compared with the supine position waveform WAs. Thus, the falling slope in the inhalation period P₁ of the prone position waveform WAp is smaller than the falling slope in the inhalation period P₁ of the supine position waveform WAs, and the rising slope in the exhalation period P₃ of the prone position waveform WAp is larger than the rising slope in the exhalation period P₃ of the supine position waveform WAs. Such a change in waveform is caused by the fact that, when the user U is in the prone position, the chest is pressed against the bed surface by his/her own weight, and thus a load is applied to the movement for inhalation and reduces the speed of inhalation. The lateral position waveform WAr has an intermediate shape between the supine position waveform WAs and the prone position waveform WAp.

The lying posture determination unit 324 determines the lying posture of the user U, based on the change in the mode of the fluctuation of the load signal s₁ depending on the lying posture of the user U, as described above. Specifically, for example, a component of the fluctuation according to the respiration of the user U is extracted from the load signal s₁ by filtering or the like, and from the length of the inhalation period P₁, the length of the post-inhalation hold period P₂, the length of the exhalation period P₃, and the like in the fluctuation, the lying posture of the user U is determined.

Note that the determination of the lying posture can be performed using other known methods. Specifically, for example, the method described in JP 2018-15210 A may be used.

### Acquisition of Biological Information

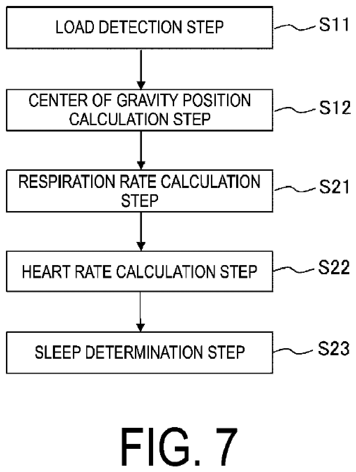

The acquisition of biological information is executed by the biological information acquisition unit 33 of the control unit 30. The biological information acquisition unit 33 of the present embodiment continuously acquires the biological information of the user U on the bed 500, at a predetermined cycle. As illustrated in the flowchart of FIG. 7, the acquisition of biological information executed by the biological information acquisition unit 33 mainly includes a load detection step S11, a center of gravity position calculation step S12, a respiration rate calculation step S21, a heart rate calculation step S22, and a sleep determination step S23.

The load detection step S11 and the center of gravity position calculation step S12 are respectively the same as the load detection step S11 and the center of gravity position calculation step S12 in the acquisition of the body information. That is, in the present embodiment, the results of the load detection step S11 and the center of gravity position calculation step S12 are used in both the acquisition of body information and the acquisition of biological information.

In the respiration rate calculation step S21, the respiration rate calculation unit 331 calculates the respiration rate of the user U. Specifically, for example, frequency analysis is performed on any of the load signals s₁ to s₄, and the respiration rate of the user U is estimated based on the value of a frequency peak in a band of about 0.2 to 0.33 Hz being a band corresponding to the frequency of human respiration.

In the heart rate calculation step S22, the heart rate calculation unit 332 calculates the heart rate of the user U. Specifically, for example, frequency analysis is performed on any of the load signals s₁ to s₄, and the heart rate of the user U is estimated based on the value of a frequency peak in a band of about 0.5 to 3.3 Hz being a band corresponding to the frequency of human heartbeat.

In the sleep determination step S23, the sleep determination unit 333 determines wakefulness/sleep of the user U.

The sleep determination unit 333 calculates an activity index ACI being a time integral value of a simple average of the standard deviations ₁ to ₄ of the load signals s₁ to s₄, by the following (Equation 3).
[Mathematical Expression 3] $ACI = {\int }_{0}^{20} \left(\frac{{σ}_{1} + {σ}_{2} + {σ}_{3} + {σ}_{4}}{4}\right) dt$ (EQUATION 3)

The integration time is 20 seconds in this case, but is not limited to 20 seconds. Since the standard deviations ₁ to ₄ increase depending on body movement of the user U, the activity index ACI increases when the user U performs body movement causing a larger load change for a longer time. That is, the activity index ACI is a parameter reflecting both the magnitude of the body movement and the duration (continuance) of the body movement.

The sleep determination unit 333 calculates a new activity index ACI every 20 seconds using the values of the standard deviations ₁ to ₄ in a respective sampling time for the past 20 seconds. Then, it is determined whether the user U is in a sleep state or a wakeful state, based on the comparison between the calculated activity index ACI and a threshold value TH_{ACI}. Specifically, for example, when the activity index ACI is smaller than the threshold value TH_{ACI}, it is determined that the user U is in the sleep state.

### Movement Control of Bed Plate

In the present embodiment, control of movement of the bed plate 520 includes a wake-up movement control (an example of "upward movement control") and a falling-asleep movement control (an example of "downward movement control"). In the wake-up movement control, the bed movement control unit 34 moves the first bed plate 521 in the horizontal position (an example of an "initial position"), to the back-up position. As a result, the user U on the bed plate 520 is prompted to wake up. In the falling-asleep movement control, the bed movement control unit 34 moves the first bed plate 521 in the back-up position to the horizontal position, and moves the third bed plate 523 and the fourth bed plate 524 in the knee-up position to the horizontal position. As a result, the shape of the bed plate 520 is changed from a shape suitable for the user U to fall asleep to a shape suitable for the user U to continue sleeping.

### Wake-up Movement Control

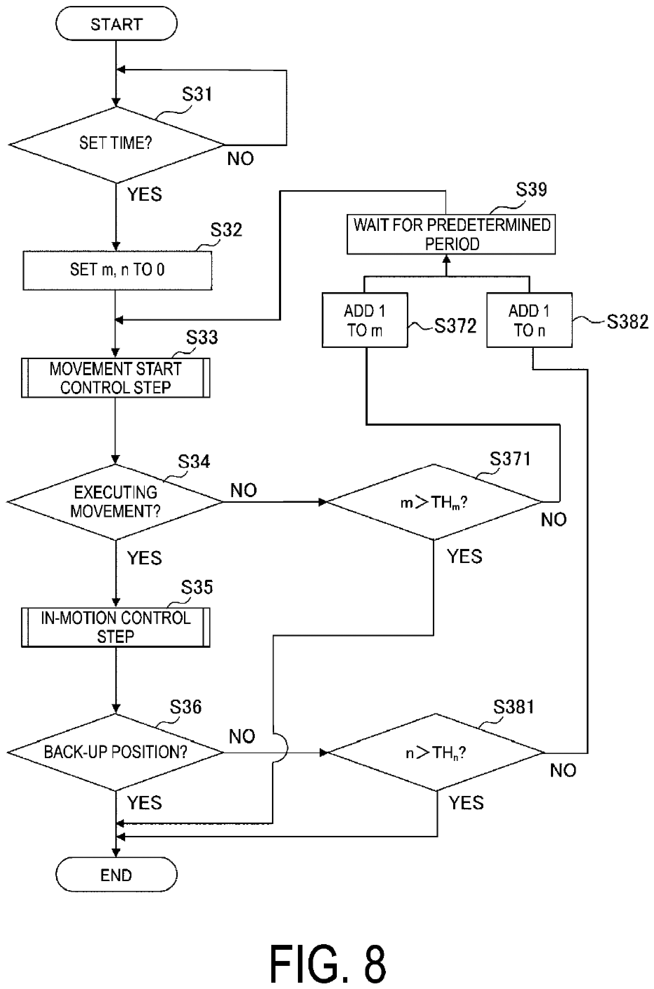

The wake-up movement control will be described by using the flowcharts of FIGS. 8, 9, and 10. In the present embodiment, the bed movement control unit 34 performs the wake-up movement control based on the information acquired in the body information acquisition step continuously executed by the body information acquisition unit 32 in parallel with the wake-up movement control.

As illustrated in FIG. 8, in step S31, the bed movement control unit 34 determines whether the current time has reached a set time. The set time is, for example, a scheduled wake-up time input in advance by the user U via the input unit 70. When the current time has not reached the set time (S31: NO), the bed movement control unit 34 executes step S31 again. When the current time has reached the set time (S31: YES), the bed movement control unit 34 sets variables m and n to "0" (step S32), and executes a movement start control step S33. Note that the variable m is a variable for counting the number of repetitions of the movement start control step S33, and the variable n is a variable for counting the number of times of stopping an in-motion control step S35 described later.

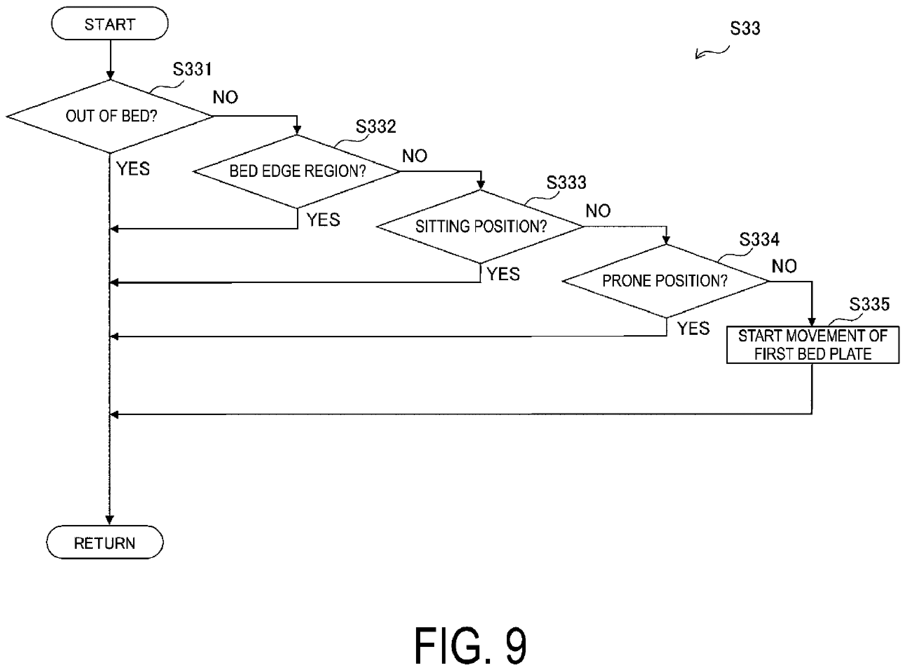

As illustrated in FIG. 9, in the movement start control step S33, the bed movement control unit 34 first executes step S331, and determines whether the user U is out of the bed plate 520. The bed movement control unit 34 refers to the latest determination result of the out-of-bed determination step S13.

When the user U is out of the bed plate 520 (S331: YES), the bed movement control unit 34 ends the movement start control step S33 without starting movement of the first bed plate 521. When the user U is present at the bed plate 520 (S331: NO), the bed movement control unit 34 determines whether the user U is located at the bed edge region BE (step S332).

In particular, for example, the bed movement control unit 34 determines whether the center of gravity G of the user U calculated in the center of gravity position calculation step S12 is located at the bed edge region BE (an example of an "edge part region"). The bed movement control unit 34 determines that the user U is located at the bed edge region BE when the center of gravity G of the user U is located at the bed edge region BE, and determines that the user U is not located at the bed edge region BE when the center of gravity G of the user U is not located at the bed edge region BE.

When the user U is located at the bed edge region BE (S332: YES), the bed movement control unit 34 ends the movement start control step S33 without starting movement of the bed plate 521. When the user U is not located at the bed edge region BE (S332: NO), the bed movement control unit 34 determines whether the user U is in the sitting position (i.e., in the sitting-up posture or the edge sitting posture) (step S333). The bed movement control unit 34 refers to the latest determination result of the posture determination step S15.

When the user U is in the sitting position (S333: YES), the bed movement control unit 34 ends the movement start control step S33 without starting movement of the first bed plate 521. When the user U is not in the sitting position (S333: NO), the bed movement control unit 34 determines whether the user U is in the prone position (step S334). The bed movement control unit 34 refers to the latest determination result of the lying posture determination step S16.

When the user U is in the prone position (S334: YES), the bed movement control unit 34 ends the movement start control step S33 without starting movement of the first bed plate 521. When the user U is not in the prone position (S334: NO), the bed movement control unit 34 operates the first movement mechanism 531 to start movement of the first bed plate 521 toward the back-up position (step S335).

Ending the movement start control step S33 without starting movement of the first bed plate 521 in the movement start control step S33 corresponds to restricting movement of the first bed plate 521 and thus the bed plate 520.

As illustrated in FIG. 8, in step S34, the bed movement control unit 34 determines whether movement of the first bed plate 521 is being executed (i.e., whether the first movement mechanism 531 is operating), following the movement start control step S33. When movement of the first bed plate 521 is not being executed (S34: NO), the bed movement control unit 34 determines whether the variable m is larger than a threshold value THₘ (step S371). When the variable m is less than or equal to the threshold value THₘ (S371: NO), the bed movement control unit 34 adds 1 to the variable m (step S372), waits for a predetermined period (step S39), and executes the movement start control step S33 again. When the variable m is larger than the threshold value THₘ (S371: YES), the bed movement control unit 34 ends the wake-up movement control. At this time, the bed movement control unit 34 may display (provide a notification) that the wake-up movement control has been ended without completing back raising of the first bed plate 520, via the display unit 50 and/or the notification unit 60.

When determining that movement of the first bed plate 521 is being executed (S34: YES), the bed movement control unit 34 executes the in-motion control step S35.

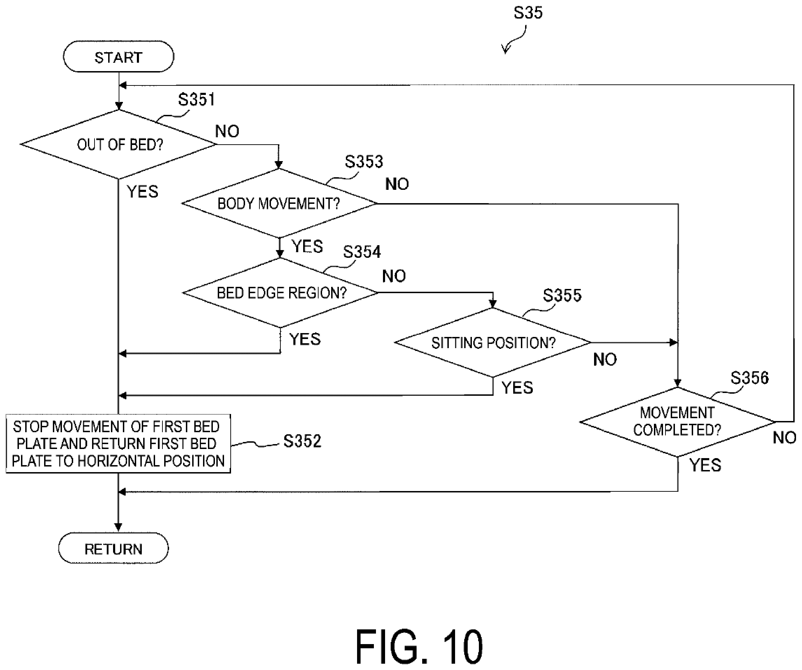

As illustrated in FIG. 10, in the in-motion control step S35, the bed movement control unit 34 first executes step S351 and determines whether the user U is out of the bed plate 520. The bed movement control unit 34 refers to the latest determination result of the out-of-bed determination step S13.

When the user U is out of the bed plate 520 (S351: YES), the bed movement control unit 34 stops the movement of the first bed plate 521, returns the first bed plate 521 to the horizontal position (step S352), and ends the in-motion control step S35. When the user U is present at the bed plate S520 (S351: NO), the bed movement control unit 34 determines whether body movement of the user U is occurring (step S353). The bed movement control unit 34 refers to the latest determination result of the body movement determination step S14.

When no body movement occurs in the user U (S353: NO), the bed movement control unit 34 determines whether the movement of the first bed plate 521 to the back-up position is completed (step S356). In the present embodiment, the bed movement control unit 34 estimates the position of the first bed plate 521 based on the operation time of the first movement mechanism 531 and the moving speed of the first bed plate 521 stored in advance. When determining that the movement of the first bed plate 521 to the back-up position is completed (S356: YES), the bed movement control unit 34 ends the in-motion control step S35. When determining that the movement of the first bed plate 521 to the back-up position is not completed (S356: NO), the bed movement control unit 34 executes step S351 again.

When body movement of the user U is occurring (S353: YES), the bed movement control unit 34 determines whether the user U is located at the bed edge region BE (step S354). The bed movement control unit 34 performs the determination in the same manner as in step S332 of the movement start control step S33. When the user U is located at the bed edge region BE (S354: YES), the bed movement control unit 34 stops the movement of the first bed plate 521, returns the first bed plate 521 to the horizontal position (step S352), and ends the in-motion control step S35.

When the user U is not located at the bed edge region BE (S354: NO), the bed movement control unit 34 determines whether the user U is in the sitting position (i.e., in the sitting-up posture or the edge sitting posture) (step S355). The bed movement control unit 34 refers to the latest determination result of the posture determination step S15.

When the user U is in the sitting position (S355: YES), the bed movement control unit 34 stops the movement of the first bed plate 521, returns the first bed plate 521 to the horizontal state (step S352), and ends the in-motion control step S35. When the user U is not in the sitting position (S355: NO), the bed movement control unit 34 determines whether the movement of the first bed plate 521 to the back-up position is completed (step S356). The bed movement control unit 34 ends the in-motion control step S35 when determining that the movement of the first bed plate 521 to the back-up position is completed (S356: YES), and executes step S351 again when determining that the movement of the first bed plate 521 to the back-up position is not completed (S356: NO).

Stopping the movement of the first bed plate 521 in the in-motion control step S35 corresponds to restricting movement of the first bed plate 521 and thus the bed plate 520.

As illustrated in FIG. 8, after the in-motion control step S35, in step S36, the bed movement control unit 34 determines whether the first bed plate 521 is at the back-up position. When the first bed plate 521 is at the back-up position (S36: YES), the bed movement control unit 34 ends the wake-up movement control.

When the first bed plate 521 is not in the back-up position (S36: NO), the bed movement control unit 34 determines whether the variable n is larger than a threshold value THₙ (step S381). When the variable n is less than or equal to the threshold value THₙ (S381: NO), the bed movement control unit 34 adds "1" to the variable n (step S382), waits for a predetermined period (step S39), and executes the movement start control step S33 again. When the variable n is larger than the threshold value THₙ (step S381: YES), the bed movement control unit 34 ends the wake-up movement control. At this time, the bed movement control unit 34 may display (provide a notification) that the wake-up movement control has been ended without completing back raising of the first bed plate 520, via the display unit 50 and/or the notification unit 60.

### Falling-asleep Movement Control

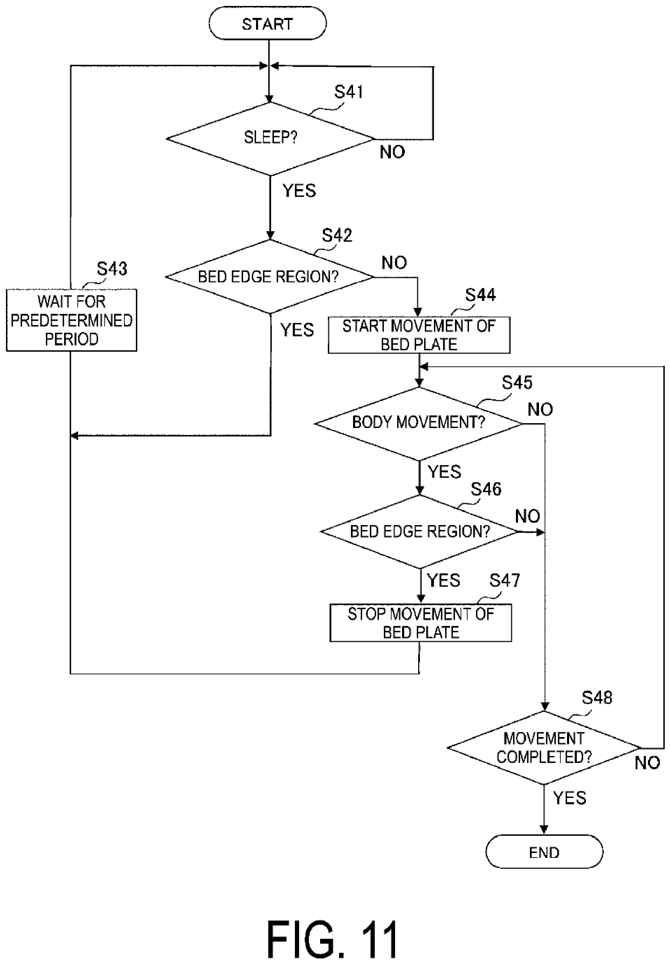

The falling-asleep movement control will be described by using the flowchart of FIG. 11. In the present embodiment, the bed movement control unit 34 performs the falling-asleep movement control based on the information acquired in the body information acquisition step continuously executed by the body information acquisition unit 32 in parallel with the falling-asleep movement control and the information acquired in the biological information acquisition step continuously executed by the biological information acquisition unit 33 in parallel with the falling-asleep movement control.

In step S41, the bed movement control unit 34 determines whether the user U is in the sleep state. The bed movement control unit 34 refers to the latest determination result of the sleep determination step S23. When the user U is not in the sleep state (S41: NO), the bed movement control unit 34 executes step S41 again.

When the user U is in the sleep state (S41: YES), the bed movement control unit 34 determines whether the user U is located at the bed edge region BE (step S42). The bed movement control unit 34 performs the determination in the same manner as in step S332 of the wake-up movement control. When the user U is located at the bed edge region BE (S42: YES), the bed movement control unit 34 waits for a predetermined period without starting movement of the first bed plate 521, the third bed plate 523, and the fourth bed plate 524 (step S43), and then executes step S41 again. Note that, not starting the movement of the first bed plate 521, the third bed plate 523, and the fourth bed plate 524 corresponds to restricting movement of the first bed plate 521, the third bed plate 523, and the fourth bed plate 524 and thus the bed plate 520.

When the user U is not located at the bed edge region BE (S42: NO), the bed movement control unit 34 operates the first movement mechanism 521 and the second movement mechanism 522 to start movement of the first bed plate 521, the third bed plate 523, and the fourth bed plate 524 toward the horizontal position (step S44).

Following step S44, the bed movement control unit 34 determines whether body movement of the user U is occurring (step S45). The bed movement control unit 34 refers to the latest determination result of the body movement determination step S14.

When body movement of the user U is occurring (S45: YES), the bed movement control unit 34 determines whether the user U is located at the bed edge region BE (step S46). The bed movement control unit 34 performs the determination in the same manner as in step S332 of the wake-up movement control. When the user U is located at the bed edge region BE (S46: YES), the bed movement control unit 34 stops the movement of the first bed plate 521, the third bed plate 523, and the fourth bed plate 524 (step S47), waits for a predetermined period (step S43), and then executes step S41 again. Note that, stopping movement of the first bed plate 521, the third bed plate 523, and the fourth bed plate 524 in step S47 corresponds to restricting movement of the first bed plate 521, the third bed plate 523, and the fourth bed plate 524, and thus movement of the bed plate 520.

When no body movement occurs in the user U (S45: NO) or when the user U is not located at the bed edge region BE (S46: NO), the bed movement control unit 34 determines whether the movement of the first bed plate 521, the third bed plate 523, and the fourth bed plate 524 to the horizontal position is completed (step S48). In the present embodiment, the bed movement control unit 34 estimates the positions of the first bed plate 521, the third bed plate 523, and the fourth bed plate 524, based on the operation times of the first movement mechanism 531 and the second movement mechanism 532, and the moving speeds of the first bed plate 521, the third bed plate 523, and the fourth bed plate 524 stored in advance. When determining that the movement of the first bed plate 521, the third bed plate 523, and the fourth bed plate 524 to the horizontal position is completed (S48: YES), the bed movement control unit 34 ends the falling-asleep movement control. When determining that the movement of the first bed plate 521, the third bed plate 523, and the fourth bed plate 524 to the horizontal position is not completed (S48: NO), the bed movement control unit 34 executes step S45 again.

Advantageous effects of the bed plate movement control system 100 of the present embodiment are summarized below.

The bed plate movement control system 100 of the present embodiment refers to the body information of the user U, in the wake-up movement control and the falling-asleep movement control. Further, when the body information satisfies a restriction condition, movement of the bed plate 520 is restricted. Specifically, in the wake-up movement control, movement of the bed plate 520 is restricted when the user U is out of bed or in the sitting-up posture (i.e., when the body information indicates that the bed plate 520 does not need to be moved), when the user U is located at the bed edge part region BE or when the user U is in the edge sitting position (i.e., when there is a risk that the user U falls from the bed plate 520), and the like. Thus, according to the bed plate movement control system 100, the bed plate 520 can be moved more suitably.

In the wake-up movement control, when determining that the lying posture of the user U is the prone position, the bed plate movement control system 100 according to the present embodiment restricts movement of the first bed plate 521 to the back-up position. Thus, according to the bed plate movement control system 100, it is possible to restrict the movement of the bed plate 520 in the direction causing physical stress in the user U, and thus, it is possible to more suitably move the bed plate 520.

After restricting movement of the first bed plate 521 in the in-motion control step S35 of the wake-up movement control, the bed plate movement control system 100 according to the present embodiment returns the first bed plate 521 to the horizontal position. Then, movement of the first bed plate 521 to the back-up position is attempted a predetermined number of times. Since the first bed plate 521 is returned to the horizontal position once in this manner, the moving distance when the first bed plate 521 is moved to the back-up position again is long, and the effect of prompting the user U to wake up is large. Further, since the movement of the first bed plate 521 to the back-up position is attempted the predetermined number of times, even when movement of the first bed plate 521 is restricted, there is a high possibility that the movement of the first bed plate 521 to the back-up position is achieved, and there is a high possibility that promotion of waking up of the user U is achieved.

After restricting movement of the bed plate 520 in the falling-asleep movement control, the bed plate movement control system 100 of the present embodiment attempts movement of the bed plate 520 again. Thus, the first bed plate 521, the third bed plate 523, and the fourth bed plate 524 can be moved to the horizontal position in a favorable manner.

### Modifications

In the embodiment described above, the following modified aspects can also be used.

In the wake-up movement control executed by the bed plate movement control system 100 of the above embodiment, whether the condition (restriction condition) for restricting movement of the first bed plate 521 is satisfied is determined in consideration of all of the position of the user U on the bed 500 (bed plate 520), the presence or absence of body movement of the user U, the posture of the user U, and the lying posture of the user U. However, no such limitation is intended. In the wake-up movement control, the bed plate movement control system 100 may determine whether the condition for restricting movement of the first bed plate 521 is satisfied based on at least one of the position of the user U on the bed plate 520, presence or absence of body movement of the user U, the posture of the user U, or the lying posture of the user U (i.e., the position and/or presence or absence of body movement and/or the posture and/or the lying posture). Note that in the present specification and the present invention, the information indicating whether the user U is out of the bed plate 520 (support surface) is included in the information indicating the position of the user U on the bed plate 520 (support surface).

Specifically, for example, the bed movement control unit 34 may determine whether the restriction condition is satisfied based on only the position of the user U on the bed plate 520. In this case, for example, in the movement start control step S33 and/or the in-motion control step S35, when the user U is located at the bed edge region BE, the bed movement control unit 34 may restrict movement of the bed plate 521 regardless of presence or absence of body movement of the user U, the posture of the user U, and the lying posture of the user U.

As another example, the bed movement control unit 34 may determine whether the restriction condition is satisfied based on the position of the user U on the bed plate 520 and presence or absence of body movement of the user U on the bed plate 520. In this case, for example, in the movement start control step S33 and/or the in-motion control step S35, when the user U is located at the bed edge region BE and body movement of the user U is occurring, the bed movement control unit 34 may restrict movement of the bed plate 521 regardless of the posture of the user U and the lying posture of the user U. Alternatively, the bed movement control unit 34 may determine whether the restriction condition is satisfied based on the position of the user U on the bed plate 520 and the lying posture of the user U on the bed plate 520. In this case, for example, in the movement start control step S33 and/or the in-motion control step S35, when the user U is located at the bed edge region BE and the user U is in the lateral position, the bed movement control unit 34 may restrict movement of the bed plate 521.

In the wake-up movement control executed by the bed plate movement control system 100 of the above-described embodiment, when movement of the first bed plate 521 is restricted in the movement start control step S33 (S34: NO) and when movement of the first bed plate 521 to the back-up position has not been completed in the in-motion control step S35 (S36: NO), the bed plate movement control system 100 executes the movement start control step S33 again. That is, repetitive control to execute the wake-up movement control again when the wake-up movement control has not been completed is performed. However, no such limitation is intended. The bed plate movement control system 100 may not execute such repetitive control in the wake-up movement control. Note that step S39 of waiting for the predetermined period may be omitted.

In the wake-up movement control executed by the bed plate movement control system 100 of the above-described embodiment, the bed plate movement control system 100 moves only the first bed plate 521. However, no such limitation is intended. In the wake-up movement control executed by the bed plate movement control system 100, the first bed plate 521 may be moved to the back-up position, and at the same time, the third bed plate 523 and the fourth bed plate 524 may be moved to the knee-up position.

In the wake-up movement control executed by the bed plate movement control system 100 of the above-described embodiment, in step S352 of the in-motion control step S35, the bed plate movement control system 100 stops the movement of the first bed plate 521, and then returns the first bed plate 521 to the horizontal position. However, limitation to this configuration is not intended, and only stopping the movement of the first bed plate 521 may be performed. In this case, movement of the first bed plate 521 after the restriction condition is released is started from the stop position at the time of the restriction of the movement of the first bed plate 521.

In the falling-asleep movement control executed by the bed plate movement control system 100 of the above-described embodiment, whether the condition (restriction condition) for restricting movement of the first bed plate 521, the third bed plate 523, and the fourth bed plate 524 is satisfied is determined in consideration of the position of the user U on the bed 500 (bed plate 520) and presence or absence of body movement of the user U. However, no such limitation is intended. In the falling-asleep movement control, the bed plate movement control system 100 may determine whether the condition for restricting the movement of the first bed plate 521, the third bed plate 523, and the fourth bed plate 524 is satisfied, based on at least one of the position of the user U on the bed plate 520, presence or absence of body movement of the user U, the posture of the user U, or the lying posture of the user U. As the restriction condition, the restriction condition in the wake-up movement control can be used as appropriate.

In the falling-asleep movement control executed by the bed plate movement control system 100 of the above-described embodiment, the bed movement control unit 34 determines whether the user U is in the sleep state in step S41, but is not limited to this configuration. The bed movement control unit 34 may determine whether the current time has reached the set time in step S41.

In the falling-asleep movement control executed by the bed plate movement control system 100 of the above-described embodiment, the bed plate movement control system 100 moves the first bed plate 521, the third bed plate 523, and the fourth bed plate 524 to the horizontal position. However, no such limitation is intended. In the falling-asleep movement control executed by the bed plate movement control system 100, only the first bed plate 521, or only the third bed plate 523, the fourth bed plate 524 may be moved to the horizontal position. At this time, the bed plate not to be moved may be in a horizontal position from the beginning. The position of the bed plate before movement in the falling-asleep movement control may be optionally settable by the user U. In addition, the bed plate movement control system 100 may not execute the repetitive control including step S43 in the falling-asleep movement control. In this case, when determining that the user U is located at the bed edge region BE in step S42 (step S42: YES), or after movement of the bed plate 520 is stopped in step S47, the falling-asleep movement control may be ended. Note that the repetitive control may be performed without step S43 of waiting for a predetermined period.

In the bed plate movement control system 100 of the above-described embodiment, the bed movement control unit 34 automatically starts the movement control of the bed plate 520. In the present specification and the present invention, "automatically starting the movement control" by the control unit means that the control unit autonomously starts the movement control based on condition determination or the like. Starting, by the control unit, the movement control of the bed plate based on determination of whether a preset condition is satisfied (for example, determination of whether a set time has been reached, and determination of whether the body information and/or the biological information of the user satisfies a predetermined condition) or the like, is included in "automatically starting the movement control" by the control unit. On the other hand, starting, by the control unit, movement of the bed plate in response to a start instruction from a human such as a user (for example, a start instruction input via a key input, a voice input, a touch panel input, or the like), is not included in "automatically starting the movement control" by the control unit. Note that the bed movement control unit 34 is not limited to the configuration of automatically starting the movement control of the bed plate 520.

The bed plate movement control system 100 of the above-described embodiment may be configured such that the user U or the like can input a desired restriction condition via the input unit 70 or the like to set a control condition to be followed by the bed movement control unit 34.

In each of the wake-up movement control and the falling-asleep movement control executed by the bed plate movement control system 100 of the above-described embodiment, whether to limit movement of the bed plate 520 may be determined based on a temporal history of at least one of the position of the user U on the bed plate 520, presence or absence of body movement of the user U, the posture of the user U, or the lying posture of the user U.

In particular, for example, when determining that the center of gravity G of the user U is at the bed edge region BE in step S332 of the wake-up movement control and/or step S42 of the falling-asleep movement control, the bed movement control unit 34 further determines whether the center of gravity G has been in a stable state for a predetermined period immediately before the time point of that determination. Specifically, for example, the bed movement control unit 34 determines whether the total movement amount of the center of gravity G in a predetermined period immediately before the time point of determination that the center of gravity G is at the bed edge region BE, is equal to or less than a predetermined distance. Then, when the total movement amount of the center of gravity G in the predetermined period is equal to or less than the predetermined distance, the position of the user U is regarded as not being at the bed edge region BE. In other words, despite the center of gravity G of the user U being at the bed edge region BE, the risk of falling is regarded to be small and movement of the bed plate 520 is allowed if the position of the center of gravity G is stable.

As another example, when determining that the lying posture of the user U is the prone position in step S334 of the wake-up movement control, the bed movement control unit 34 further determines whether the user U has continued the prone position beyond a predetermined period immediately before the time point of that determination. Then, when the user U has continued the prone position beyond the predetermined period, the user U is regarded as not being in the prone position. In other words, when the user U has continued the prone position beyond the predetermined period, prompting the user U to wake up is prioritized over not causing physical stress in the user U.

In the bed plate movement control system 100 of the above embodiment, the restriction of movement of the bed plate 520 is the stop of movement of the bed plate 520, but is not limited to the stop of movement of the bed plate 520. The restriction of movement of the bed plate 520 may be to limit the moving speed of the bed plate 520 to a low speed (i.e., a speed lower than the moving speed when the movement of the bed plate 520 is not restricted).

In each of the wake-up movement control and the falling-asleep movement control executed by the bed plate movement control system 100 of the above embodiment, movement of the bed plate 520 may be restricted in a first mode when at least one of the position of the user U on the bed plate 520, presence or absence of body movement of the user U, the posture of the user U, or the lying posture of the user U satisfies a first condition, and movement of the bed plate 520 may be restricted in a second mode different from the first mode when at least one of the position of the user U on the bed plate 520, presence or absence of body movement of the user U, the posture of the user U, or the lying posture of the user U satisfies a second condition different from the first condition.

Specifically, for example, the bed edge region BE is divided into a first bed edge region located at an edge part of the bed plate 520 in the width direction and a second bed edge region inside the first bed edge region. Further, in the wake-up movement control and the falling-asleep movement control, movement of the bed plate 520 is stopped when the user U is located at the first bed edge region, and the bed plate 520 is moved at a low speed (i.e., a speed lower than the moving speed when the movement of the bed plate 520 is not restricted) when the user U is located at the second bed edge region. In this case, the "restriction" when the user U is located at the first bed edge region is "stop", and the "restriction" when the user U is located at the second bed edge region is "speed limitation".

As another example, the "restriction" when the user U is located at the bed edge region BE and body movement of the user U is occurring may be "stop", and the "restriction" when the user U is located at the bed edge region BE and no body movement occurs in the user U may be "speed limitation".

In restricting movement of the bed plate 520 in the bed plate movement control system 100 of the above embodiment, only movement of the first bed plate 521 may be restricted when the center of gravity G of the user U is located at a head-side edge part region being a region within a predetermined distance from the edge part of the bed plate 520 at the head side (the positive side in the Y direction), and only movement of the third bed plate 523 and the fourth bed plate 524 may be restricted when the center of gravity G of the user U is located at a leg-side edge part region being a region within a predetermined distance from the edge part of the bed plate 520 at the leg side (the negative side in the Y direction). As a result, only movement of the bed plate 520 in the region where the user U is located can be restricted.

In the bed plate movement control system 100 of the above-described embodiment, information indicating the position, presence or absence of body movement, the posture, and/or the lying posture of the user U may be acquired based on an image captured by an image capturing apparatus.

In the bed plate movement control system 100 of the above embodiment, any biological signal acquisition unit for acquiring the biological signal of the user U (a signal fluctuating with the biological activity of the user U) can be used instead of the load detection unit 10. Specifically, for example, a plurality of pressure-sensitive sensors (pressure sensors) arranged in a matrix form under a bed sheet can acquire fluctuation in the pressure applied by the user U to the bed plate 520 as a biological signal. In this aspect, the position of the center of gravity, presence or absence of body movement, the posture, the lying posture, and the like of the user can be obtained based on outputs of the plurality of pressure-sensitive sensors.

The bed plate movement control system 100 of the above embodiment need not necessarily include all of the load detectors 11 to 14, but may only include one of these detectors. For example, when three load detectors are used, the position of the center of gravity of the user U on the top surface of the bed plate 520 can be detected as long as the load detectors are not arranged in a straight line. The load detectors do not necessarily need to be arranged at the four corners of the bed and can be arranged at any position such that the load of the user on the bed and the fluctuation of the load can be detected. As the load detectors 11 to 14, for example, a force sensor can be used without being limited to a load sensor using a beam-type load cell.

In the above embodiment, the case of the bed plate movement control system 100 being used for the bed 500 has been described as an example, but the bed plate movement control system 100 is not necessarily used in the bed 500. In any support equipment, such as a bed, a sofa, a chair, or a wheelchair, including a support surface being for supporting a human body and configured to be movable, the bed plate movement control system 100 can be used as a support surface movement control system for controlling movement of the support surface of the support equipment. In this case, the support surface movement control system may be capable of executing an upward movement control for moving a part of the support surface (a backrest, a leg rest, or the like) in a direction of increasing the inclination angle of the part with respect to the horizontal plane and/or a decrease movement control for moving the part in a direction of decreasing the inclination angle of the part with respect to the horizontal plane.

As long as the features of the present invention are maintained, the present invention is not limited to the embodiment described above, and other forms considered within the scope of the technical concept of the present invention are also included within the scope of the present invention.

### Reference Signs List

11, 12, 13, 14 Load detector; 30 Control unit; 31 Center of gravity position calculation unit; 32 Body information acquisition unit; 321 Out-of-bed determination unit; 322 Body movement determination unit; 323 Posture determination unit; 324 Lying posture determination unit; 33 Biological information acquisition unit; 34 Bed movement control unit; 500 Bed; 520 Bed plate

## Claims

1. A support surface movement control device for controlling movement of a support surface supporting a body of a user, the support surface movement control device comprising:
a body information acquisition unit configured to acquire body information of the user on the support surface; and
a movement control unit configured to automatically start and execute movement control to move the support surface,
wherein, in the movement control, the movement control unit restricts movement of the support surface when the body information satisfies a restriction condition.

2. The support surface movement control device according to claim 1, wherein the body information includes at least one of a position, presence or absence of body movement, a posture, or a lying posture of the user on the support surface.

3. The support surface movement control device according to claim 1 or 2, wherein, in the movement control, the movement control unit restricts the movement of the support surface when a position of the user is within an edge part region on the support surface.

4. The support surface movement control device according to any one of claims 1 to 3, wherein, in the movement control, the movement control unit restricts the movement of the support surface, when a position of the user is within an edge part region on the support surface and body movement of the user on the support surface occurs.

5. The support surface movement control device according to any one of claims 1 to 4, wherein
the movement control includes an upward movement control to move a part of the support surface in a direction of increasing an inclination angle of the part with respect to a horizontal plane, and
in the upward movement control, the movement control unit restricts the movement of the support surface when the lying posture of the user on the support surface is a prone position.

6. The support surface movement control device according to any one of claims 1 to 5, wherein the body information includes temporal history of at least one of a position, presence or absence of body movement, a posture, or a lying posture of the user on the support surface.

7. The support surface movement control device according to any one of claims 1 to 6, wherein, in the movement control, the movement control unit restricts the movement of the support surface in a first mode when the body information satisfies a first condition, and restricts the movement of the support surface in a second mode different from the first mode when the body information satisfies a second condition different from the first condition.

8. The support surface movement control device according to any one of claims 1 to 7, wherein
the movement control includes an upward movement control to move a part of the support surface, from an initial position, in a direction of increasing an inclination angle of the part with respect to a horizontal plane, and
in the upward movement control, when the body information satisfies the restriction condition, the movement control unit stops movement of the part and returns the part to the initial position.

9. The support surface movement control device according to claim 8, wherein, in the upward movement control, the movement control unit
determines whether the body information satisfies the restriction condition, after returning the part to the initial position, and
moves the part in a direction of increasing an inclination angle of the part with respect to a horizontal plane, when the body information does not satisfy the restriction condition.

10. The support surface movement control device according to any one of claims 1 to 9, wherein,
the movement control includes a downward movement control to move a part of the support surface in a direction of decreasing an inclination angle of the part with respect to a horizontal plane, and
in the downward movement control, the control device
stops movement of the part when the body information satisfies the restriction condition,
determines whether the body information satisfies the restriction condition, after stopping the movement of the part, and
moves the part in a direction of decreasing an inclination angle of the part with respect to a horizontal plane, when the body information does not satisfy the restriction condition.

11. Support equipment comprising:
a support surface configured to support a body of a user; and
the support surface movement control device according to any one of claims 1 to 10.
